# FASCICULE DE BREVET EUROPEEN

(11) **EP 2 709 980 B1**
(45) Date de publication et mention de la délivrance du brevet: **04.04.2018**
(21) Numéro de dépôt: 12719990.9
(22) Date de dépôt: 14.05.2012
(51) Int. Cl.: C07C 51/44, C07C 51/48, C07C 53/08, B01D 3/14, B01D 3/40, B01D 11/04

(54) **PROCEDE DE RECUPERATION D'ACIDE ACETIQUE**
VERFAHREN ZUR RÜCKGEWINNUNG VON ESSIGSÄURE
METHOD FOR RECOVERING ACETIC ACID

(30) Priorité: 17.05.2011 FR 1154265
(43) Date de publication de la demande: 26.03.2014
(73) Titulaire: Rhodia Acetow GmbH, 79108 Freiburg (DE)
(72) Inventeur: AMOROS, Daniel, 30570 Valleraugue (FR); BREHELIN, Mathias, 69003 Lyon (FR); HUMMEL, Andreas, 79108 Freiburg (DE); KRUMREY, Thomas, 79331 Teningen (DE)
(74) Mandataire: Trinks, Ole
(86) Numéro de dépôt international: PCT/EP2012/058914
(87) Numéro de publication internationale: WO 2012/156362

(56) Documents cités:
- WO-A1-03/074781
- US-A1- 2009 234 157
- VAN DUC LONG N ET AL: "Design and optimization of a dividing wall column for debottlenecking of the acetic acid purification process", CHEMICAL ENGINEERING AND PROCESSING, ELSEVIER SEQUOIA, LAUSANNE, CH, vol. 49, no. 8, 1 août 2010 (2010-08-01), pages 825-835, XP027234377, ISSN: 0255-2701 [extrait le 2010-08-01]

## Description

La présente invention concerne un procédé de récupération d'acide acétique. L'invention concerne plus particulièrement un procédé comprenant une étape d'extraction liquide-liquide d'une solution aqueuse contenant de l'acide acétique, et une étape de distillation de l'extrait obtenu.

L'acide acétique, l'anhydride acétique et l'acide peracétique sont utilisés dans les industries de chimie organique, par exemple pour la fabrication d'acétate de cellulose, d'acétates d'alkyles, de cétènes, de glycérine et d'acides epoxyalcanoïques, et des solutions contenant de l'acide acétique sont extraites de ces procédés.

Par exemple, de l'acide acétique est formé comme sous-produit à une concentration de 20 à 40% dans le procédé de fabrication d'acétate de cellulose, et de l'acide acétique est formé comme sous-produit à une concentration de 10 à 15% dans le procédé de fabrication de glycérine de par l'utilisation d'acide peracétique. Des solutions aqueuses contenant de l'acide acétique à une telle concentration moyenne comprise entre environ 7 et environ 40% sont produites en grande quantité comme sous-produits, et une récupération efficace de l'acide acétique de ces solutions aqueuses est indispensable et importante pour améliorer la performance économique des procédés principaux. De plus, l'acide acétique est utilisé dans d'autres domaines, par exemples dans les industries de traitement des métaux et dans les industries de fermentation, et également dans ces domaines, des solutions aqueuses contenant de l'acide acétique sont formées. Afin d'améliorer le taux d'utilisation de substances précieuses et de prévenir la pollution environnementale, il est très important de récupérer l'acide acétique de ces solutions aqueuses avec une grande efficacité.

Des méthodes de récupération d'acide acétique de solutions aqueuses sont connues. Une de ces méthodes consiste à réaliser une extraction liquide-liquide avec un solvant de bas point d'ébullition. L'acide acétique se retrouve dans la phase solvant. Puis l'acide acétique, le solvant, un peu d'eau dissoute, et éventuellement des composés de point d'ébullition élevé, qui constituent la phase solvant, sont récupérés, généralement par distillation. Cette purification par distillation de nombreux composés peut s'avérer très complexe et très coûteuse à mettre en oeuvre. US 2009/234157 A1 divulgue un procédé de purification de flux contenant de l'acide acétique comprenant une étape d'extraction liquide-liquide, optionnellement suivie d'une étape de distillation. WO 03/074781 divulgue un procédé de purification de flux contenant de l'acide acétique comprenant une étape de distillation dans une colonne à cloison. N. Van Duc Long et al., Chemical Engineering and Processing, vol. 49, N° 8, pages 825 - 835, divulguent l'utilisation de colonnes à cloison et de colonnes Petyluk dans les procédés de purification de l'acide acétique sans l'associer à une étape d'extraction liquide-liquide.

Par ailleurs, dans le domaine de la purification par distillation, des colonnes à cloison et des colonnes de « Petlyuk » ont été développées. Ces colonnes permettent de limiter le nombre de colonnes à mettre en oeuvre dans le procédé de purification, par rapport à un procédé de purification avec des colonnes classiques, ce qui simplifie le procédé de purification, et ce qui assure une réduction de la consommation d'énergie.

On est toujours à la recherche de procédés simples et peu coûteux de récupération d'acide acétique, et qui permettent l'obtention de composés de pureté élevée.

A cet effet, l'invention propose un procédé de récupération d'acide acétique comprenant une étape d'extraction liquide-liquide d'une solution aqueuse contenant de l'acide acétique avec un solvant de bas point d'ébullition, pour extraire l'acide de la solution aqueuse, et une étape de distillation de cet extrait, caractérisé en ce que la distillation est réalisée dans une colonne à cloison ou dans une colonne « Petlyuk ».

La solution aqueuse contenant de l'acide acétique introduite dans l'étape d'extraction de l'invention comprend avantageusement entre 20 et 40% en poids d'acide acétique. L'acide acétique peut être issu d'une matière renouvelable d'origine animale ou végétale.

Selon un mode de réalisation particulier du procédé de l'invention, cette solution aqueuse contenant de l'acide acétique est issue d'un procédé d'acétylation de la pâte de bois.

Généralement, un procédé d'acétylation de pâte de bois comprend les étapes de mise en contact de pâte de bois avec de l'acide acétique, de réaction avec l'anhydride acétique pour acétyler la cellulose. Le produit obtenu est le triacétate de cellulose, qui est ensuite hydrolysé avec de l'eau et un catalyseur pour former l'acétate-2,5 de cellulose. Une solution aqueuse contenant entre 20 et 40% en poids d'acide acétique est récupérée dans la section suivante de précipitation et lavage du produit à l'eau.

Selon ce mode de réalisation, la solution aqueuse contenant l'acide acétique comprend généralement des composés de point d'ébullition élevé. Le procédé de l'invention permet de séparer de façon efficace ces composés de l'acide acétique. On peut citer à titre d'exemples de composés de point d'ébullition élevé les sucres, les sels tels que NaHSO₄ ou CaSO₄, les hémicelluloses etc.

Selon ce mode de réalisation, l'acide acétique récupéré lors de l'étape de distillation avec une colonne à cloison ou avec une colonne « Petlyuk », dans au moins une fraction intermédiaire, est recyclé dans le procédé d'acétylation du bois. L'acide acétique récupéré dans la(les) fraction(s) intermédiaire(s) peut être sous forme pratiquement pure ou sous forme de solution aqueuse concentrée, par exemple comprenant de l'ordre de 60% en poids d'acide acétique.

Le procédé de l'invention comprend une étape d'extraction liquide-liquide de la solution aqueuse contenant de l'acide acétique avec un solvant de bas point d'ébullition, pour extraire l'acide de la solution aqueuse.

Le solvant de bas point d'ébullition a une température d'ébullition inférieure à celle de l'acide acétique.

Le solvant est avantageusement choisi dans le groupe des éthers, des alcools, des acétates ou des cétones.

De préférence le solvant est choisi parmi le diéthyl éther, le méthyl tertio butyl éther, l'alcool isopropylique, l'acétate isopropylique, l'acétate d'éthyle, l'acétate de méthyle et la méthyl éthyl cétone.

L'étape d'extraction peut être réalisée dans de nombreux appareils dans lesquels deux phases liquides sont mises en contact l'une avec l'autre. Pour une extraction efficace, il est préférable de choisir un appareil dans lequel l'interface entre les deux phases liquides est fréquemment « rafraîchie ». Dans l'industrie chimique, on utilise habituellement, comme appareils d'extraction en continu, des colonnes à garnissage ordonné ou à garnissage vrac, ou des colonnes à plateaux perforés. On peut aussi utiliser une cascade de mélangeurs décanteurs.
Pour améliorer le mélange entre les phases solvant et aqueuse, des dispositifs d'extraction en colonnes agitées peuvent être utilisées comme les colonnes disque/couronne de type RDC (« rotary disc contractor »), les colonnes agitées de type Kuhni .

De préférence, le contact entre la solution contenant de l'acide acétique à extraire et le solvant de bas point d'ébullition est réalisé à contre-courant.

Avantageusement la température lors de l'étape d'extraction est inférieure ou égale à 50°C. La pression lors de cette étape est de préférence la pression atmosphérique.

La phase solvant issue de l'extraction (extrait) contient principalement de l'acide acétique et du solvant, un peu d'eau dissoute, et éventuellement des composés de point d'ébullition élevé.

Le procédé de l'invention comprend également une étape de distillation dans une colonne à cloison ou dans une colonne « Petlyuk », de l'extrait issu de l'étape d'extraction.

Le but de cette distillation dans une colonne à cloison ou dans une colonne « Petlyuk » est la séparation et la récupération efficace des différents composés de l'extrait, à savoir le solvant d'extraction, l'acide acétique, et éventuellement les composés de point d'ébullition élevé.

Généralement on récupère :
- en tête de colonne, le solvant d'extraction et éventuellement un peu d'eau
- dans la partie latérale de la colonne, au moins une fraction intermédiaire ; la(les) fraction(s) intermédiaire(s) comprend (comprennent) de l'acide acétique sous forme pratiquement pure ou de l'acide acétique sous forme de solution aqueuse concentrée
- en pied de colonne des composés de point d'ébullition élevé, seuls ou en mélange avec de l'acide acétique

Par « colonne à cloison », on entend une colonne dont une partie de la colonne est séparée verticalement en deux parties par une cloison. La présence de la cloison génère une section de préfractionnement située dans la zone d'alimentation.

La colonne « Petlyuk » est une alternative à la colonne à cloison. Il s'agit d'une colonne dans laquelle la section de préfractionnement de la colonne à cloison est remplacée par une colonne séparée de préfractionnement, dont le produit de tête est introduit dans la partie haute de la colonne suivante (qui n'est pas une colonne à cloison), et dont le produit de pied est introduit dans la partie basse de cette colonne. La colonne de préfractionnement n'a pas de bouilleur ou de condenseur, à cloison, mais du liquide de la partie haute de la colonne suivant la colonne de préfractionnement, et de la vapeur de la partie basse de cette colonne suivante, sont introduits dans la colonne de préfractionnement.

La colonne à cloison de l'invention comprend au moins une cloison. De préférence elle contient une seule cloison.

La cloison peut être une plaque métallique soudée à la colonne, qui peut par exemple être d'une épaisseur d'environ 1,5 mm. Il peut également s'agir d'une cloison non soudée à la virole, et solidaire des internes de la colonne.

La cloison est de préférence située au milieu de la colonne, de part et d'autre de l'endroit où le flux d'alimentation est introduit dans la colonne, et de part et d'autre de l'endroit où la(les) fraction(s) intermédiaire(s) est (sont) récupéré(es).

Les dimensions de la colonne à cloison peuvent être très variables. Son diamètre peut par exemple varier entre 0,3 et plus de 5 m. Sa hauteur peut aller jusqu'à 100 m.

Avantageusement la pression de la colonne est la pression atmosphérique.

De préférence, le domaine de température entre la tête de colonne et le pied de colonne est compris entre 20 et 130°C.

La(les) fraction(s) intermédiaire(s) récupérée(s) comprend(comprennent) avantageusement entre 50 et 100% en poids d'acide acétique.

Avantageusement, le solvant d'extraction récupéré en tête de colonne, lors de la distillation, est recyclé dans l'étape d'extraction. En tête de colonne, le solvant d'extraction peut contenir un peu d'eau, il peut par exemple comprendre entre 0 et 10% en poids d'eau. Aussi, avant d'être recyclé dans l'étape d'extraction, l'eau peut être séparée du solvant, par exemple par simple décantation.

Le procédé de l'invention comprenant une étape d'extraction et une étape de distillation avec une colonne à cloison ou une colonne « Petlyuk », permet de récupérer l'acide acétique et le solvant d'extraction avec une pureté élevée. Le procédé est simple et très intéressant économiquement. En utilisant une colonne à cloison ou une colonne « Petlyuk », on peut réduire le nombre de colonnes (et donc réduire le nombre d'enveloppes de colonne, de bouilleurs, de condenseurs, d'internes), ce qui réduit considérablement les coûts d'investissement de l'installation de distillation et sa complexité. Il permet aussi une réduction significative de la consommation d'énergie.

La figure 1 représente le schéma d'un procédé conventionnel de récupération d'acide acétique.
La figure 2 représente le schéma d'un mode de réalisation du procédé de l'invention.
La figure 3 représente le schéma d'un autre mode de réalisation du procédé de l'invention.

D'autres détails ou avantages de l'invention apparaîtront plus clairement à la vue des exemples donnés ci-dessous.

### EXEMPLES

### Exemple 1 (comparatif)

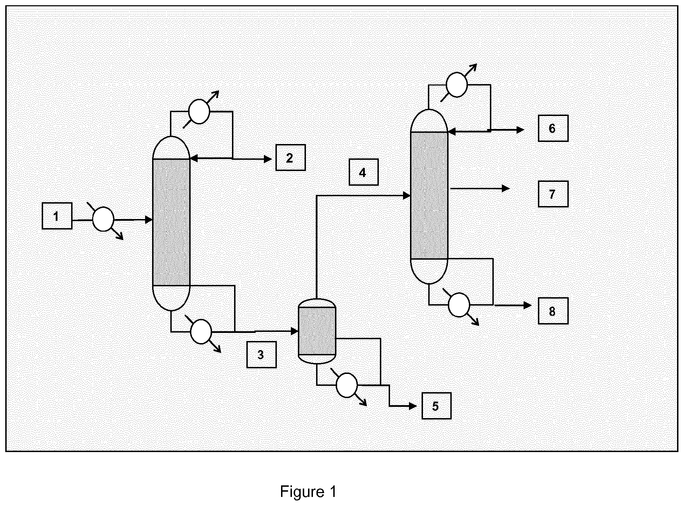

La figure 1 représente le schéma d'un procédé conventionnel de récupération d'acide acétique.

A l'issue du procédé d'acétylation de la cellulose, on réalise l'étape d'extraction d'une solution aqueuse d'acide acétique comprenant 30% en poids d'acide acétique. Le solvant d'extraction est le diéthyl éther. L'extraction est réalisée à 25°C et à pression atmosphérique, dans une colonne à plateaux perforés.

Le flux 1 représente le produit extrait par le solvant provenant de l'étape d'extraction. Le flux 1 est composé d'un mélange eau, solvant, acide acétique et composés de point d'ébullition élevé. Le flux 1 est introduit dans une colonne de distillation. Le flux 2 est le distillat sortant en tête de colonne, il est composé majoritairement de solvant qui est recyclé dans l'étape d'extraction. Le flux 3 sortant en pied de la colonne est introduit dans un ballon de flash. Ce flash sépare le flux 3 en 2 autres flux : le flux 4 contenant un mélange d'eau et d'acide acétique avec une proportion de 75% en poids d'acide acétique, et le flux 5 contenant la majorité des composés de point d'ébullition élevé. Le flux 4 alimente la colonne de purification d'acide acétique. En tête de colonne on extrait un mélange 6 riche en eau comprenant de 70 à 90% en poids d'eau, qui est renvoyé à l'étape d'extraction. Le flux 7, constitué d'un mélange d'eau et d'acide acétique dans une proportion allant de 35 à 45 % d'eau est soutiré sur la partie latérale de cette colonne; ce flux est recyclé vers le procédé d'acétylation de la cellulose. Le flux 8 qui est soutiré dans le fond de colonne, est de l'acide acétique pur (99,5%), qui est recyclé vers le procédé d'acétylation de la cellulose.

### Exemple 2

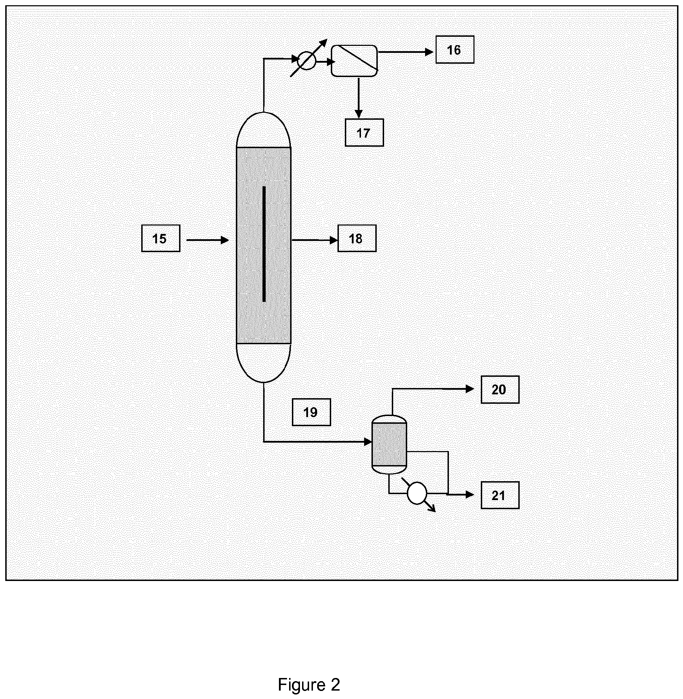

La figure 2 représente le schéma d'un mode de réalisation du procédé de l'invention, comprenant une colonne à cloison et un ballon de flash.

Le flux 15 représente le produit extrait par le solvant provenant de l'étape d'extraction (mêmes conditions pour l'étape d'extraction que pour l'exemple 1 comparatif). Le flux 15 est introduit dans une colonne à cloison. Les flux 16 et 17 sont issus d'une décantation à une température comprise entre 5 et 40 °C et préférentiellement 20°C du flux sortant en tête de colonne à cloison. Le flux 16 contient majoritairement le solvant qui est recyclé vers l'étape d'extraction et le flux 17 contient majoritairement de l'eau. Le flux 18 est soutiré dans la zone cloisonnée opposée au flux 15 et est constitué d'un mélange d'acide acétique et d'eau comprenant entre 50% et 70 % en poids d'acide acétique. Ce flux 18 est recyclé vers la section réactionnelle du procédé d'acétylation de la cellulose. Le flux 19 est extrait en pied de colonne à cloison et contient de l'acide acétique et des composés de point d'ébullition élevé. Ce flux alimente un ballon de flash. Ce flash sépare le flux 19 en 2 autres flux: le flux 20 contenant l'acide acétique pur, qui est recyclé vers la section réactionnelle du procédé d'acétylation de la cellulose, et le flux 21 contenant des composés de point d'ébullition élevé et de l'acide acétique.

On décrit ci-dessous un exemple de réalisation du procédé selon ce mode de réalisation.

Le tableau 1 ci-dessous décrit les compositions des différents flux ainsi que les conditions opératoires . Les pourcentages mentionnés dans le tableau sont des pourcentages en poids.

**Tableau 1**

| Flux | 15 | 18 | 19 | 21 | 20 | 16 | 17 |
|---|---|---|---|---|---|---|---|
| Acide acétique (%) | 15,82 | 57,65 | 98,76 | 49,98 | 99,92 | 0,12 | 0,33 |
| Eau (%) | 6,91 | 42,35 | 0,08 | 0,02 | 0,08 | 1,08 | 95,63 |
| Solvant(%) | 77,14 | - | - | - | - | 98,80 | 4,04 |
| Composés de point d'ébullition élevé (%) | 0,13 | - | 1,16 | 50,00 | - | - | - |
| Débit (kg/h) | 38751,6 | 3200,0 | 4300,0 | 100,0 | 4200 | 30211,4 | 1040,2 |
| Température (°C) | 50 | 102 | 118 | 118 | 118 | 25 | 25 |

La zone de distillation située au-dessus de la zone cloisonnée a une efficacité correspondant à 12 étages théoriques.

La zone de distillation située au-dessous de la zone cloisonnée où se fait le soutirage latéral 18 a une efficacité correspondant à 15 étages théoriques.

La zone de distillation située dans la partie gauche de la zone cloisonnée où se fait l'alimentation de la colonne par le flux 15 a une efficacité correspondant à 12 étages théoriques.

La zone de distillation située dans la partie droite de la zone cloisonnée où se fait le soutirage latéral du flux 18 a une efficacité correspondant à 8 étages théoriques.

La colonne est opérée à pression atmosphérique et le taux de reflux en tête de colonne est de 1.

Le dispositif de colonne à cloison permet de séparer avec un seul appareil : le solvant qui est recyclé vers l'étape d'extraction, un mélange acide acétique et eau qui est recyclé vers le procédé d'acétylation de la cellulose, l'acide acétique pur contenant des composés de point d'ébullition élevé qui sont à séparer dans un ballon de flash.

### Exemple 3

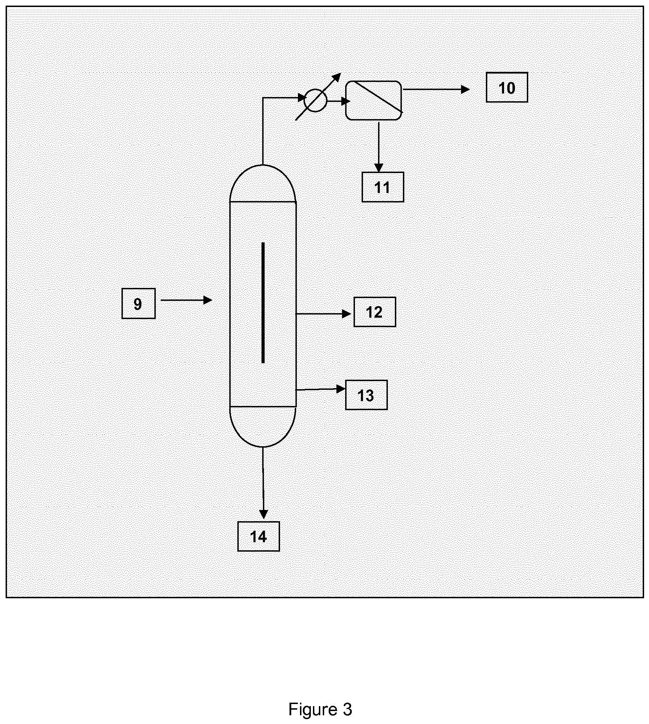

La figure 3 représente le schéma d'un mode de réalisation du procédé de l'invention, comprenant une colonne à cloison.

Le flux 9 représente le produit extrait par le solvant provenant de l'étape d'extraction (mêmes conditions pour l'étape d'extraction que pour l'exemple 1 comparatif). Il est composé d'un mélange eau, solvant, acide acétique et composés de point d'ébullition élevé. Le flux 9 est introduit dans la colonne à cloison. Le flux de tête de colonne a une température comprise entre 30 et 35 °C et est composée d'un mélange eau, solvant comprenant environ 4% en poids d'eau. Ce flux alimente un décanteur qui a une température comprise entre 5 et 40 °C et préférentiellement 20°C. Les flux 10 et 11 sont issus de cette décantation. Le flux 10 contient majoritairement le solvant qui est recyclé vers l'étape d'extraction et le flux 11 contient majoritairement de l'eau. Le flux 12 est soutiré dans la zone cloisonnée opposée au flux 9 et est constitué d'un mélange d'acide acétique et d'eau comprenant entre 50% et 70 % en poids d'acide acétique. Ce flux 12 est recyclé vers la section réactionnelle du procédé d'acétylation de la cellulose. Le flux 13 est soutiré au-dessous de la zone cloisonnée de la colonne et est constitué d'acide acétique pur. Ce flux 13 est recyclé vers la section réactionnelle du procédé d'acétylation de la cellulose. Le flux 14 est soutiré en pied de la colonne à cloison et contient des composés de point d'ébullition élevé et de l'acide acétique.

On décrit ci-dessous un exemple de réalisation du procédé selon ce mode de réalisation.

Le tableau 2 ci-dessous décrit les compositions des différents flux ainsi que les conditions opératoires . Les pourcentages mentionnés dans le tableau sont des pourcentages en poids.

**Tableau 2**

| Flux | 9 | 12 | 13 | 14 | 10 | 11 |
|---|---|---|---|---|---|---|
| Acide acétique (%) | 15,82 | 57,65 | 99,92 | 49,98 | 0,12 | 0,33 |
| Eau (%) | 6,91 | 42,35 | 0,08 | 0,02 | 1,08 | 95,63 |
| Solvant(%) | 77,14 | - | - | - | 98,80 | 4,04 |
| Composés de point d'ébullition élevé (%) | 0,13 | - | - | 50,00 | - | - |
| Débit (kg/h) | 38751,6 | 3200,0 | 4200,0 | 100,0 | 30211,4 | 1040,2 |
| Température (°C) | 50 | 102 | 118 | 118 | 25 | 25 |

La zone de distillation située au-dessus de la zone cloisonnée a une efficacité correspondant à 12 étages théoriques.

La zone de distillation située au-dessous de la zone cloisonnée où se fait le soutirage latéral 13 a une efficacité correspondant à 15 étages théoriques.

La zone de distillation située dans la partie gauche de la zone cloisonnée où se fait l'alimentation de la colonne par le flux 9 a une efficacité correspondant à 12 étages théoriques.

La zone de distillation située dans la partie droite de la zone cloisonnée où se fait le soutirage latéral du flux 12 a une efficacité correspondant à 8 étages théoriques.

La colonne est opérée à pression atmosphérique et le taux de reflux en tête de colonne est de 1.

Le dispositif de colonne à cloison permet de séparer avec un seul appareil : le solvant qui est recyclé vers l'étape d'extraction, un mélange acide acétique et eau qui est recyclé vers le procédé d'acétylation de la cellulose, l'acide acétique pur qui est également recyclé vers le procédé d'acétylation de la cellulose, et les composés de point d'ébullition élevé qui sont détruits.

## Revendications

1. Procédé de récupération d'acide acétique comprenant une étape d'extraction liquide-liquide d'une solution aqueuse contenant de l'acide acétique avec un solvant de bas point d'ébullition, pour extraire l'acide de la solution aqueuse, et une étape de distillation de cet extrait, **caractérisé en ce que** la distillation est réalisée dans une colonne à cloison ou dans une colonne Petlyuk.

2. Procédé selon la revendication 1, **caractérisé en ce que** la solution aqueuse comprend entre 20 et 40% en poids d'acide acétique.

3. Procédé selon la revendication 1 ou 2, **caractérisé en ce que** la solution aqueuse est issue d'un procédé d'acétylation de la pâte de bois.

4. Procédé selon la revendication 3, **caractérisé en ce que** la solution aqueuse comprend également des composés de point d'ébullition élevé.

5. Procédé selon la revendication 4, **caractérisé en ce que** l'acide acétique récupéré , lors de l'étape de distillation avec une colonne à cloison ou avec une colonne Petlyuk, dans au moins une fraction intermédiaire, est recyclé dans le procédé d'acétylation de la pâte de bois.

6. Procédé selon l'une des revendications précédentes, **caractérisé en ce que** le solvant de bas point d'ébullition est choisi dans le groupe des éthers, des alcools, des acétates ou des cétones.

7. Procédé selon la revendication 6, **caractérisé en ce que** le solvant de bas point d'ébullition est choisi parmi le diéthyl éther, le méthyl tertio butyl éther, l'alcool isopropylique, l'acétate isopropylique, l'acétate d'éthyle, l'acétate de méthyle et la méthyl éthyl cétone.

8. Procédé selon l'une des revendications précédentes, **caractérisé en ce que** la pression de la colonne est la pression atmosphérique.

9. Procédé selon l'une des revendications précédentes, **caractérisé en ce que** le domaine de température entre la tête de colonne et le pied de colonne est compris entre 20 et 130°C.

10. Procédé selon la revendication selon l'une des revendications précédentes, **caractérisé en ce que** le solvant de bas point d'ébullition est récupéré après décantation du mélange solvant/eau sortant en tête de colonne.

11. Procédé selon la revendication selon l'une des revendications précédentes, **caractérisé en ce que** le solvant de bas point d'ébullition est recyclé dans l'étape d'extraction.

## Patentansprüche

1. Verfahren zur Rückgewinnung von Essigsäure, umfassend einen Schritt der Flüssig-/Flüssig-Extraktion einer essigsäurehaltigen, wässrigen Lösung mit einem Lösungsmittel mit niedrigem Siedepunkt, um die Säure aus der wässrigen Lösung zu extrahieren, und einen Schritt der Destillation dieses Extrakts, **dadurch gekennzeichnet, dass** die Destillation in einer Trennwandkolonne oder in einer Petlyuk-Kolonne erfolgt.

2. Verfahren nach Anspruch 1, **dadurch gekennzeichnet, dass** die wässrige Lösung zwischen 20 und 40 Gew.-% Essigsäure enthält.

3. Verfahren nach Anspruch 1 oder 2, **dadurch gekennzeichnet, dass** die wässrige Lösung aus einem Verfahren der Acetylierung von Holzstoff stammt.

4. Verfahren nach Anspruch 3, **dadurch gekennzeichnet, dass** die wässrige Lösung auch Verbindungen mit hohem Siedepunkt enthält.

5. Verfahren nach Anspruch 4, **dadurch gekennzeichnet, dass** die beim Schritt der Destillation mit einer Trennwandkolonne oder einer Petlyuk-Kolonne gewonnene Essigsäure in zumindest einer Zwischenfraktion bei dem Verfahren zur Acetylierung von Holzstoff wiederverwertet wird.

6. Verfahren nach einem der vorangehenden Ansprüche, **dadurch gekennzeichnet, dass** das Lösungsmittel mit niedrigem Siedepunkt ausgewählt ist aus der Gruppe der Ether, Alkohole, Acetate oder Ketone.

7. Verfahren nach Anspruch 6, **dadurch gekennzeichnet, dass** das Lösungsmittel mit niedrigem Siedepunkt ausgewählt ist aus Diethylether, Methyltertiärbutylether, Isopropuylalkohol, Isopropylacetat, Ethylacetat, Methylacetat und Methylethylketon.

8. Verfahren nach einem der vorangehenden Ansprüche, **dadurch gekennzeichnet, dass** der Druck der Kolonne der Atmosphärendruck ist.

9. Verfahren nach einem der vorangehenden Ansprüche, **dadurch gekennzeichnet, dass** der Temperaturbereich zwischen dem Kolonnenkopf und dem Kolonnenfuß zwischen 20 und 130°C beträgt.

10. Verfahren nach einem der vorangehenden Ansprüche, **dadurch gekennzeichnet, dass** das Lösungsmittel mit niedrigem Siedepunkt nach Dekantieren des Gemischs aus Lösungsmittel/Wasser rückgewonnen wird, das am Kolonnenkopf austritt.

11. Verfahren nach einem der vorangehenden Ansprüche, **dadurch gekennzeichnet, dass** das Lösungsmittel mit niedrigem Siedepunkt bei dem Schritt der Extraktion rückgewonnen wird.

## Claims

1. Process for the recovery of acetic acid comprising a stage of liquid/liquid extraction of an aqueous solution comprising acetic acid with a low-boiling-point solvent, in order to extract the acid from the aqueous solution, and a stage of distillation of this extract, **characterized in that** the distillation is carried out in a dividing-wall column or in a Petlyuk column.

2. Process according to Claim 1, **characterized in that** the aqueous solution comprises between 20% and 40% by weight of acetic acid.

3. Process according to Claim 1 or 2, **characterized in that** the aqueous solution results from a process for the acetylation of wood pulp.

4. Process according to Claim 3, **characterized in that** the aqueous solution also comprises high-boiling-point compounds.

5. Process according to Claim 4, **characterized in that** the acetic acid recovered during the stage of distillation with a dividing-wall column or with a Petlyuk column, in at least one intermediate fraction, is recycled in the process for the acetylation of wood pulp.

6. Process according to one of the preceding claims, **characterized in that** the low-boiling-point solvent is chosen from the group of the ethers, alcohols, acetates and ketones.

7. Process according to Claim 6, **characterized in that** the low-boiling-point solvent is chosen from diethyl ether, methyl tert-butyl ether, isopropyl alcohol, isopropyl acetate, ethyl acetate, methyl acetate and methyl ethyl ketone.

8. Process according to one of the preceding claims, **characterized in that** the pressure of the column is atmospheric pressure.

9. Process according to one of the preceding claims, **characterized in that** the temperature range between the column top and the column bottom is between 20 and 130°C.

10. Process according to claim according to one of the preceding claims, **characterized in that** the low-boiling-point solvent is recovered after separation by settling of the solvent/water mixture exiting at the column top.

11. Process according to claim according to one of the preceding claims, **characterized in that** the low-boiling-point solvent is recycled in the extraction stage.
